(19) Europäisches Patentamt
European Patent Office
Office européen des brevets

(11) Numéro de publication : **0 659 755 A1**

(12) **DEMANDE DE BREVET EUROPEEN**

(21) Numéro de dépôt : **94402975.0**

(22) Date de dépôt : **21.12.94**

(51) Int. Cl.$^6$ : **C07F 9/10,** A61K 7/48, C12P 9/00, C07F 9/117, C07K 7/06, C07K 5/08, C07K 5/10

(30) Priorité : **27.12.93 FR 9315683**

(43) Date de publication de la demande : **28.06.95 Bulletin 95/26**

(84) Etats contractants désignés : **AT BE CH DE DK ES FR GB GR IE IT LI LU NL PT SE**

(71) Demandeur : **ROUSSEL-UCLAF**
**35, Boulevard des Invalides**
**F-75007 Paris (FR)**

(72) Inventeur : **Fourneron, Jean-Dominique**
**Bât. D. Prévalmon,**
**Traverse Gauffant**
**F-13009 Marseille (FR)**
Inventeur : **Fructus, Alain**
**15, rue Carle Hébert**
**F-92400 Courbevoie (FR)**

(74) Mandataire : **Vieillefosse, Jean-Claude et al**
**ROUSSEL UCLAF,**
**111 Route de Noisy**
**F-93235 Romainville Cédex (FR)**

(54) **Phospholipides vecteurs de molécules actives, leur préparation et leur utilisation dans des compositions cosmétiques ou dermatologiques.**

(57) L'invention a pour objet les phospholipides actifs de formule (I) :

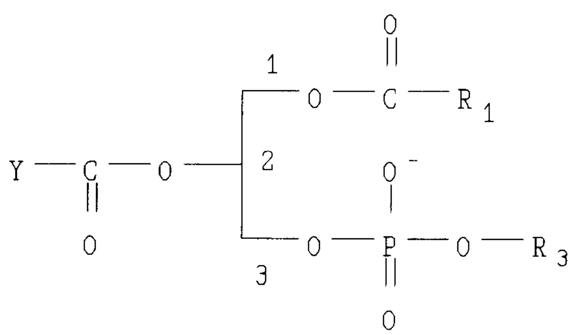

(I)

dans laquelle :
$R_1$ représente notamment une chaîne aliphatique de 14 à 24 atomes de carbone, saturée ou avec 1 ou 2 insaturations,
$R_3$ représente notamment un reste de choline, d'éthanolamine, de glycérol, de sérine, d'inositol, d'éthanol, de n-propanol, de n-butanol ou encore d'éthylène glycol,
et Y est tel que

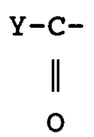

représente une molécule active en position 2 du glycérol destinée à être libérée sous l'action de phospholipases.

EP 0 659 755 A1

Les phospholipides sont très répandus dans la nature. Ils sont les constituants majeurs des parois des cellules animales et végétales. Leur propriété fondamentale est de pouvoir se constituer en "bi-couches", celles-ci constituant la structure des parois de cellules.

La constitution des phospholipides naturels est très variée, cette variété jouant des rôles importants dans les métabolismes cellulaires, rôles parfois non encore élucidés.

La structure des phospholipides est la suivante :

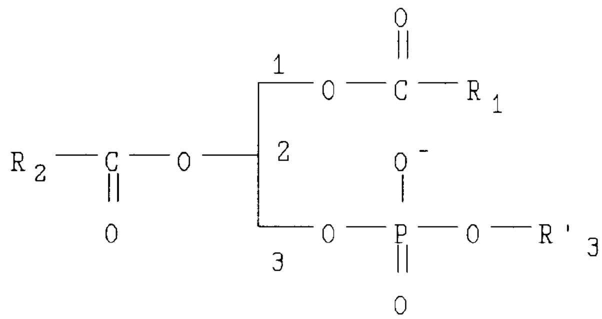

Les glycérophospholipides naturels comportent quatre fonctions esters : les fonctions esters carboxyliques

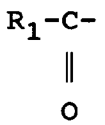 et 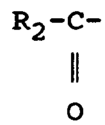

respectivement en position 1 et 2 du glycérol d'une part et entre le phosphore et la "tête polaire" variable, d'autre part, que constitue $R_3$-O-$PO_2$-O-. $R_1$ et $R_2$ identiques ou différents peuvent représenter notamment une chaîne aliphatique de 14 à 24 atomes de carbone, saturée ou avec 1 ou 2 insaturations, telle que par exemple la chaîne palmitique, oléique, linoléique, stéarique ou myristique,
$R'_3$ peut représenter notamment un reste de choline, éthanolamine, glycérol, sérine ou encore d'inositol.

Ainsi notamment deux acides gras sont estérifiés sur les positions 1 et 2 du glycérol et le 3ème hydroxyle du glycérol est estérifié par un phosphatidyl ester.

La position 2 du glycérophospholipide est particulièrement importante. En effet de nombreuses phospholipases A2 existent dans les tissus et notamment dans la peau, de façon à libérer l'acide gras correspondant,

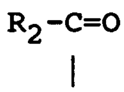

lequel peut être ensuite métabolisé dans l'organisme. Ces phospholipases A2 sont notamment décrites dans les publications suivantes : Slotta, K. H. (1960) The Enzymes 4, 552 et Van Deenen, L. L. M. & de Haas, G. H. (1966) Ann. Rev. Biochem. 35 (1), 674.

Par ailleurs, les phospholipides exogènes ont une affinité pour les tissus et particulièrement pour la peau, et sont capables de s'absorber dans les parois des cellules.

L'objet de la présente invention est d'apporter aux cellules des éléments fonctionnels par l'intermédiaire de phospholipides semi-synthétiques : en incorporant des molécules fonctionnelles en position 2 d'un phospholipide, on les rend particulièrement bio-disponibles car elles vont pénétrer rapidement les tissus puis les cellules par l'affinité de leurs résidus phospholipides avec les phospholipides des membranes et vont ensuite libérer la molécule fonctionnelle sous l'action des phospholipases A2.

La présente invention a ainsi pour objet de nouvelles molécules que sont les phospholipides actifs de formule (I) :

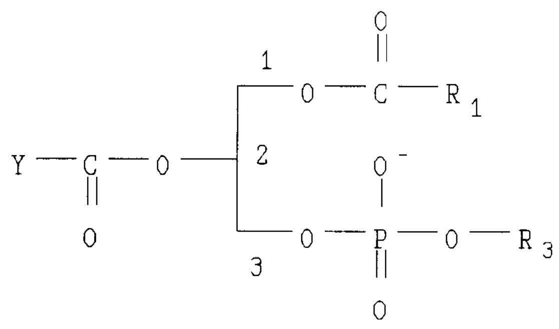

(I)

dans laquelle :

$R_1$ représente notamment une chaîne aliphatique de 14 à 24 atomes de carbone, saturée ou avec 1 ou 2 insaturations,

$R_3$ représente notamment un reste de choline, d'éthanolamine, de glycérol, de sérine, d'inositol, d'éthanol, de n-propanol, de n-butanol ou encore d'éthylène glycol,

et Y est tel que

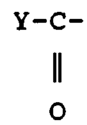

représente une molécule active en position 2 du glycérol destinée à être libérée sous l'action de phospholipases.

Par chaîne aliphatique de 14 à 24 atomes de carbone saturée ou avec 1 ou 2 insaturations, on entend notamment la chaîne palmitique, oléique, linoléique, stéarique ou myristique.

Les phospholipides de formule (I) dans laquelle $R_3$ représente un radical éthyle, propyle ou butyle, sont particulièrement intéressants comme le montrent les tests décrits dans la partie expérimentale.

La présente invention a donc particulièrement pour objet des phospholipides actifs caractérisés en ce que $R_3$ représente un radical éthyle.

La synthèse peut se faire à partir de phospholipides naturels, particulièrement ceux extraits du jaune d'oeuf à partir de jaune d'oeuf préparé industriellement ou du jaune d'oeuf du commerce, ou de soja, lécithine de soja, en 3 étapes pour aboutir au phospholipide actif de formule (I).

La présente invention a précisément pour objet les phospholipides actifs tels que définis ci-dessus, caractérisés en ce que la molécule active à greffer en position 2 est choisie parmi les molécules suivantes : la vitamine A acide, l'acide all-trans rétinoïque, l'acide 9-cis rétinoïque, l'acide 13-cis rétinoïque, les acides gras essentiels comme l'acide $\gamma$ linolénique, l'acide $\alpha$ linolénique, l'acide eicosapentaenoïque (EPA), l'acide docosahexaenoïque (DHA), les acides $\alpha$ hydroxylés comme l'acide glycolique, l'acide lactique, l'acide tartrique, l'acide $\alpha$ méthyllactique, l'acide $\alpha$ hydroxybutyrique, l'acide gluconique, l'acide mandélique, l'acide mucique, l'acide malique, l'acide $\alpha$ phényllactique, l'acide saccharique, l'acide tartronique ; les acides divers tels que l'acide kojique, l'acide asiaticoside, l'acide madécassique, l'acide benzoïque, l'acide glutamique, l'acide malonique, l'acide phytique, l'acide ascorbique, l'acide nordihydroguaiaretique, l'acide salicylique, l'acide 18$\beta$ glycyrrhétinique ; les acides aminés comme la tyrosine, l'hydroxyproline, la lysine, l'arginine, des petits peptides fonctionnels comme le pyroGlu-Glu-Asp-Ser-GlyoH ou le Gly-His-Lys ou le Arg-Gly-Asp-Ser, ou encore les monoesters de diacide tels que le succinate de farnésile, de rétinol et les monoamides de diacide de formule générale

$$HO_2\text{-}C\text{-}(CH_2)_n\text{-}CO\text{-}X\text{-}R$$

dans laquelle n représente un nombre compris entre 2 et 16, X représente un atome de soufre, d'azote ou d'oxygène et R représente un radical inclus dans la liste des acides ci-dessus.

La présente invention a plus particulièrement pour objet des phospholipides actifs, tels que définis ci-dessus, caractérisés en ce que la molécule active à greffer en position 2 est choisie parmi les molécules suivantes : la vitamine A acide, l'acide $\gamma$ linolénique, l'acide eicosapentaenoïque (EPA), l'acide docosahexaenoïque (DHA), l'acide kojique, l'acide asiaticoside, l'acide madécassique, l'acide glutamique, l'acide phytique, l'acide glycolique, l'acide lactique, l'acide ascorbique, l'acide nordihydroguaiaretique, les acides aminés comme la tyrosine, des petits peptides fonctionnels comme le pyroGlu-Glu-Asp-Ser-GlyoH ou le Gly-His-Lys ou le Arg-Gly-Asp-Ser, l'acide 18β glycyrrhétinique.

La présente invention a aussi pour objet un procédé de préparation des phospholipides actifs tels que définis ci-dessus.

D'une manière générale, la chimie des glycérophospholipides est une chimie généralement conduite dans des mélanges de solvants de façon à s'accommoder de la nature amphipatique de ces molécules. Les produits sont généralement obtenus très dilués d'où des coûts élevés. Il en découle qu'il n'existe pratiquement aucune utilisation de ces produits en tant que molécules parfaitement définies.

Le procédé de préparation des phospholipides actifs, objet de l'invention, consiste, à partir de phospholipides naturels, à intervenir successivement sur deux des liaisons esters des glycérophospholipides par remplacement de la tête polaire $R_3$-O- et ensuite par hydrolyse enzymatique sélective de la position 2.

Le procédé selon l'invention de préparation de phospholipides actifs de formule (I) telle que définie ci-dessus, est caractérisé en ce que l'on soumet un phospholipide ou un mélange de phospholipides de sources naturelles, de formule (II) :

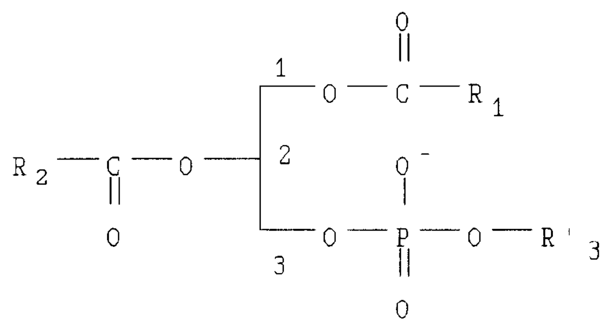

(II)

dans laquelle $R_1$ et $R_2$ identiques ou différents peuvent représenter une chaîne aliphatique de 14 à 24 atomes de carbone saturée ou avec 1 ou 2 insaturations telle que la chaîne palmitique, oléïque, linoléique, stéarique ou myristique,
et $R'_3$ représente notamment un reste de choline, d'éthanolamine, de glycérol, de sérine ou encore d'inositol, à une réaction de substitution de $R'_3$ par un radical éthyle, propyle ou butyle, au moyen d'une transphosphatidylation enzylatique pour obtenir le produit de formule (III) :

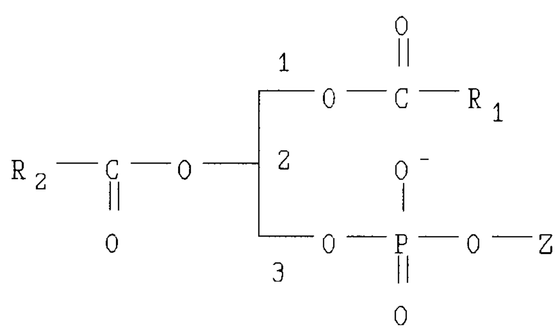

(III)

dans laquelle $R_1$ et $R_2$ ont les significations indiquées ci-dessus et Z représente un radical éthyle, propyle ou butyle, que l'on soumet à une hydrolyse par voie enzymatique de la fonction ester en position 2 du glycérol, pour obtenir un produit de formule (IV) :

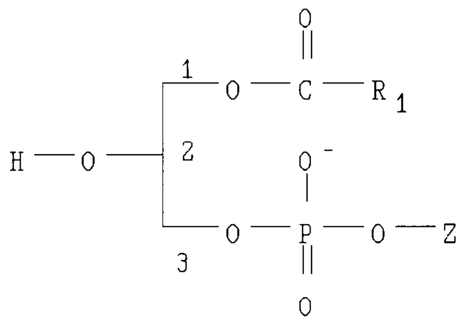

(IV)

dans laquelle $R_1$ et Z ont les significations indiquées ci-dessus, que l'on soumet à une réaction d'acylation de l'hydroxyle libéré, par un anhydride de formule (V) :

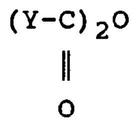

(V)

ou un anhydride mixte correspondant dans laquelle

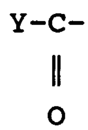

représente un reste de molécule active telle que définie ci-dessus.

Le produit de formule (I) dans laquelle Z représente un radical éthyle, propyle ou butyle, obtenu selon le procédé ci-dessus décrit, peut être transformé si désiré et si nécessaire en produit de formule (I) dans laquelle Z a la signification de $R_3$ indiquée ci-dessus, par les moyens connus de l'homme du métier.

Dans un mode de réalisation préféré du procédé ci-dessus décrit :

- les sources naturelles de phospholipides sont d'origine animale ou végétale tels que notamment le jaune d'oeuf, la lécithine végétale par exemple de soja,
- le phospholipide de source naturelle est plus particulièrement la phosphatidylcholine ou la phosphatidyléthanolamine, ou un mélange de ces phospholipides.

La phosphatidylcholine et la phosphatidyléthanolamine correspondant à un produit de formule (II) dans laquelle -O-$R'_3$ représente respectivement un reste de choline

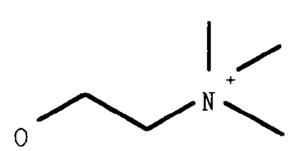

et un reste d'éthanolamine

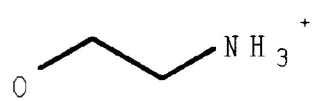

- la substitution du radical $R'_3$ par un radical éthyle, propyle ou butyle effectuée par transphosphatidylation enzymatique est réalisée au moyen de la phospholipase D en milieu éthanolique, propanolique ou butanolique (référence Enzyme Handbook, Thomas E. Barman p. 545), cette réaction étant complète

et univoque ;

- l'hydrolyse par voie enzymatique pour obtenir le lysophosphatidyl éthanol (propanol ou butanol) de formule (IV) est réalisée au moyen de la phospholipase $A_2$, enzyme utilisée non purifiée, en milieu calcique $Ca^{++}$ ;
- l'acylation de l'hydroxyle libéré dans le composé de formule (IV) par la ou les molécules biologiquement actives à transporter dans les membranes est réalisée par voie enzymatique ou chimique, après acidification du lysophosphatidyl éthanol (propanol ou butanol), en utilisant un anhydride simple ou mixte de la substance active, au sein d'un solvant tel que par exemple le diéthyléther ou le toluène.

L'originalité de cette synthèse réside dans l'étroite implication entre des étapes de chimie traditionnelle et l'utilisation de la spécificité de plusieurs enzymes lipolytiques.

La principale innovation réside principalement dans l'utilisation du phospholipide éthanol (propanol ou butanol) intermédiaire (PZ) qui intervient pour faciliter toutes les étapes de la synthèse. Les bonnes activités enzymatiques de la phospholipase A2 et de la lipase pancréatique sur les phospholipides de ce type permettent d'utiliser des enzymes brutes provenant d'une même source : la poudre pancréatique.

Le phospholipide éthanol (propanol ou butanol) est obtenu de manière univoque et la méthode de purification par précipitation que l'on utilise permet l'obtention d'un produit propre sous la forme d'un sel de calcium. L'intérêt de ce protocole par rapport à ceux décrits (Eibl et Kovatchev 1981, et Omodeo Salé, Cestaro et al. 1989) est l'obtention d'un sel de calcium du PZ facilement isolable à l'exclusion du sel de sodium qui se forme en présence d'acétate de sodium, et l'absence de produits parasites tels que l'acide phosphatidique ou les produits de départ non transformés. Par ailleurs, l'utilisation du sel de calcium dans l'étape suivante qui fait appel à une hydrolyse catalysée par la phospholipase $A_2$ ($PA_2$) strictement calcio-dépendante est beaucoup plus favorable que l'utilisation de phosphatidyléthanol (propanol ou butanol) sous forme acide.

La présente invention a plus particulièrement pour objet un procédé de préparation de phospholipides actifs tel que défini ci-dessus, caractérisé en ce que la substance active à incorporer au phospholipide en position 2 est choisie parmi les substances suivantes :
la vitamine A acide, l'acide all-trans rétinoïque, l'acide 9-cis rétinoïque, l'acide 13-cis rétinoïque, les acides gras essentiels comme l'acide $\gamma$ linolénique, l'acide $\alpha$ linolénique, l'acide eicosapentaenoïque (EPA), l'acide docosahexaenoïque (DHA), les acides $\alpha$ hydroxylés comme l'acide glycolique, l'acide lactique, l'acide tartrique, l'acide $\alpha$ méthyllactique, l'acide $\alpha$ hydroxybutyrique, l'acide gluconique, l'acide mandélique, l'acide mucique, l'acide malique, l'acide $\alpha$ phényllactique, l'acide saccharique, l'acide tartronique ; les acides divers tels que l'acide kojique, l'acide asiaticoside, l'acide madécassique, l'acide benzoïque, l'acide glutamique, l'acide malonique, l'acide phytique, l'acide ascorbique, l'acide nordihydroguaiaretique, l'acide salicylique, l'acide 18$\beta$ glycyrrhétinique ; les acides aminés comme la tyrosine, l'hydroxyproline, la lysine, l'arginine, des petits peptides fonctionnels comme le pyroGlu-Glu-Asp-Ser-GlyoH ou le Gly-His-Lys ou le Arg-Gly-Asp-Ser ou encore les monoesters de diacide tels que le succinate de farnésile, de rétinol et les monoamides de diacide de formule générale

$$HO_2\text{-}C\text{-}(CH_2)_n\text{-}CO\text{-}X\text{-}R$$

dans laquelle n représente un nombre compris entre 2 et 16, X représente un atome de soufre, d'azote ou d'oxygène et R représente un radical inclus dans la liste des acides ci-dessus,
et tout particulièrement parmi les substances suivantes : la vitamine A acide, l'acide $\gamma$ linolénique, l'acide eicosapentaenoïque (EPA), l'acide docosahexaenoïque (DHA), l'acide kojique, l'acide asiaticoside, l'acide madécassique, l'acide glutamique, l'acide phytique, l'acide glycolique, l'acide lactique, l'acide ascorbique, l'acide nordihydroguaiaretique, les acides aminés comme la tyrosine, des petits peptides fonctionnels comme le pyroGlu-Glu-Asp-Ser-GlyoH ou le Gly-His-Lys ou le Arg-Gly-Asp-Ser, l'acide 18$\beta$ glycyrrhétinique.

Parmi les produits de formule (I) tels que définis ci-dessus, on peut citer de façon non limitative à titre d'exemple, les phospholipides actifs suivants qui peuvent être vecteurs des substances actives telles que définies ci-dessus, destinés à des applications particulières.

Un phospholipide actif vecteur d'un ester de rétinol tel que la vitamine A acide ou l'un de ses isomères par exemple l'acide all-trans rétinoïque, l'acide 9-cis rétinoïque ou l'acide 13-cis rétinoïque, peut être utilisé en vue d'obtenir une activité anti-acné notamment une activité sur les comédons ou une activité anti-rides.

Un phospholipide actif vecteur d'acide $\gamma$ linolénique est utilisé pour la restauration de la fluidité des parois membranaires et pour l'amélioration de l'état des peaux de personnes atopiques pour lesquelles le manque d'acide $\gamma$ linolénique provoque des perturbations importantes au niveau de la peau.

Un phospholipide actif vecteur d'acide eicosapentaenoïque (EPA) et/ou d'acide docosahexenoïque (DHA) est utilisé pour le traitement du psoriasis car il a été montré qu'un apport exogène en acides gras améliore fortement l'état de la peau psoriasique.

Un phospholipide actif vecteur d'un $\alpha$ hydroxy acide comme par exemple l'acide glycolique, l'acide lactique, l'acide tartrique, l'acide $\alpha$ méthyl lactique, l'acide gluconique, l'acide mandelique, l'acide mucique, l'acide

malique, est utilisé pour le traitement des hyperkératoses, de l'acné, du vieillissement, notamment du vieillissement actinique.

Les acides asiaticoside et madécassique, extraits de la plante Centella Asiatica, sont connus pour avoir des propriétés cicatrisantes et anti-cellulitique. Ils incitent les fibroblastes à produire du collagène d'excellente qualité. Greffés et transportés par un phospholipide actif, leur activité est décuplée.

L'acide phytique est un excellent chelateur de métaux. Par ce fait, il possède des propriétés anti-oxydantes, anti-radicalaires et anti-métallo-enzymes, notamment anti-proteases. On peut incorporer l'acide phytique en position 2 d'un phospholipide actif et ainsi le rendre plus biodisponible.

L'acide ascorbique ou vitamine C est aussi un excellent anti-radicalaire et anti-oxydant. Il participe à toutes sortes de cycles biochimiques notamment dans la peau. Incorporé à un phospholipide actif, il est beaucoup plus stable et plus biodisponible, très utile par exemple dans le cas des irradiations solaires, où il capte le fer biologique et limite les effets néfastes de l'inflammation.

L'acide nordihydroguaiaretique est également un excellent anti-oxydant et il est utilisé pour le traitement des kératoses solaires; greffé en position 2 d'un phospholipide actif, sa biodisponibilité est considérablement augmentée ce qui permet d'utiliser des doses plus faibles pour la même activité et donc de diminuer les effets secondaires néfastes.

Un phospholipide actif porteur de tyrosine en position 2 est facilement absorbé par la peau et permet un bronzage plus intense car la synthèse de la mélanine est basée sur l'utilisation de la tyrosine.

L'acide 18β glycyrrhétinique est un très bon anti-inflammatoire mais assez peu soluble et qui pénètre difficilement dans la peau. Greffé en position 2 d'un phospholipide actif, sa pénétration dans la peau est fortement augmentée ainsi que son efficacité.

La présente invention a également pour objet des compositions cosmétiques ou dermatologiques destinées au soin et traitement de la peau et plus particulièrement aux peaux acnéiques, aux peaux déshydratées, aux peaux lésées, aux peaux ridées, caractérisées en ce qu'elles renferment des phospholipides actifs tels que définis ci-dessus de préférence selon le pourcentage de 0,1 à 10% et particulièrement de 0,4 à 5% (exprimé par rapport à la composition complète) et des excipients usuels.

La présente invention a particulièrement pour objet des compositions cosmétiques destinées notamment au soin des comédons, caractérisées en ce qu'elles renferment un phospholipe actif vecteur d'un ester de rétinol tel que la vitamine A acide ou l'un de ses isomères tels que l'acide all-trans rétinoïque, l'acide 9-cis rétinoïque.

On utilise des excipients appropriés pour apporter ces différents phospholipides actifs à la peau.

Parmi les excipients, on utilise fréquemment des agents tensio-actifs comme des esters de sorbitan tel le stéarate de sorbitan, des esters de sorbitan oxyéthylénés comme le palmitate de sorbitan POE, des esters de sucrose comme le sucrose cocoate, des esters de glucose ou de méthylglucose oxyéthylénés ou non, comme le méthylglucéth-20 ou le méthylglucose sesquistéarate, des acyl phosphates neutralisés comme le potassium cétylphosphate, des acides gras éthoxylés comme l'acide stéarique éthoxylé, les alcools gras éthoxylés comme l'alcool stéarylique éthoxylé, les lécithines plus ou moins déhuilées, d'oeuf ou de soja, hydrogénées ou non, des stérols végétaux éthoxylés.

Les excipients peuvent également contenir des mouillants, des conservateurs tels le méthylparaben, le biosol, le bronopol, des parfums, des colorants, des charges comme le talc ou le polyméthacrylate.

Eventuellement, les compositions selon l'invention peuvent également contenir des additifs insolubles ou solubles tels que les principes actifs complémentaires liposolubles ou hydrosolubles, par exemple des filtres solaires, des écrans aux radiations solaires, pour leur conférer un pouvoir protecteur vis à vis des rayonnements solaires, des extraits vitaminés et des anti-oxydants et des agents dispersants et stabilisants.

Lorsque des additifs sont insolubles dans les phases huileuses et aqueuses, ils constituent donc une phase supplémentaire. Ils sont choisis par exemple parmi les produits suivants :

- les perfluoroéthers comme les FOMBLIN (R) de la Société MONTECATINI,
- les pigments insolubles comme :
  . les oxydes de titane,
  . oxyde de titane rutile,
  . oxyde de titane anatase,
  . oxyde de titane pyrogéné comme le P 25 (R) de Degussa,
  . oxyde de titane micronisé dans le SUN VEIL (R) d'Ikeda,
  . oxyde de titane traité en surface par des silicones ou par des acides aminés, ou par de la lécithine, ou par des stéarates métalliques,
  . oxyde de fer,
  . oxyde de fer traité en surface par des silicones, ou par des acides aminés, ou par de la lécithine, ou par des stéarates métalliques,

. oxyde de zinc,
. oxyde de zinc micronisé comme l'UFZO (R) de Cosmo Trends Corporation,
. mica recouvert d'oxyde de titane.

La présente invention a également pour objet des compositions dermatologiques ou cosmétiques, telles que définies ci-dessus, caractérisées en ce qu'elles renferment un ou plusieurs principes actifs liposolubles complémentaires incorporés dans la phase grasse de l'émulsion, choisis notamment parmi les substances suivantes pour lesquelles sont indiqués ci-dessous les pourcentages préférés exprimés par rapport à la formule complète finale :

- Palmitate de vitamine A : de 500 à 10 000 UI/g.
- Filtres solaires liposolubles : octyl méthoxycinnamate : 0,5 à 10 %, isoamyl éthoxycinnamate : 0,5 à 10 %, octyl diméthyl 25 paba : 0,5 à 8 %, octyl salicylate : 0,5 à 5 %, butyl méthoxy-dibenzoyl méthane : 0,5 à 5 %, benzophénone 3 : 0,5 à 10 %, octyl triazone : 0,5 à 5 %, 4-polyéthoxy aminobenzoate d'éthyle : 0,5 à 10 %, isopropyle 4-dibenzoyl méthane : 0,5 à 5 %.
- Insaponifiables de maïs, de karité, de soja ou d'avocat : 0,1 à 3 %.
- Ximénoil (R) (mélange huileux renfermant 50% d'acide Ximénique) : 0,1 à 5 %, de l'extrait essentiel d'huile de sésame : 0,1 à 4 %, de l'huile de maïs péroxydée : 0,1 à 10 %, l'acétate de tocophérols : 0,05 à 7 %, des tocophérols naturels : 0,05 à 5 %, du farnesol : 0,05 à 5 %, de l'acide linoléique 2 à 10%.

La présente invention a aussi pour objet des compositions dermatologiques ou cosmétiques telles que décrites ci-dessus, caractérisées en ce qu'elles renferment un ou plusieurs principes actifs complémentaires hydrosolubles incorporés dans la phase aqueuse de l'émulsion, choisis notamment parmi le lactate de sodium, des extraits de biolysat d'Hafnia, des extraits de biolysat de Klebsellia pneumoniae et des filtres solaires hydrosolubles.

Les principes actifs complémentaires hydrosolubles peuvent également être choisis parmi les substances ci-dessus pour lesquelles sont indiqués les pourcentages préférés exprimés par rapport à la formule complète finale :

- Acide 2-phényl benzimidazol 5-sulfonique neutralisé : 0,5 à 8 %
- Acide 2-hydroxy 4-méthoxybenzophénone 5-sulfonique neutralisé : 0,5 à 5 %
- Acide ascorbique : 0,5 à 10 %, benzoate de caféine : 0,1 à 5 %, acide phytique : 0,1 à 5 %, acide mucique : 0,1 à 5 %, hydrolysats de protéines végétales : 0,1 à 10 %, polyglucan : 0,1 à 5 %
- Extrait de mimosa du Mexique : 0,5 à 20 %, chitosan : 0,5 à 20 %, sérum d'animaux marins : 0,1 à 3 %
- Extrait d'hirudine : 0,5 à 10 %, extrait de méristem : 0,1 à 5 %, oligomères procyanodoliques: 0,05 à 3 %, extraits de levures : 0,05 à 3 %, panthénol : 0,05 à 5 %, extrait de centella asiatica : 0,05 à 3 %, l'acide glycyrrhétinique : 0,05 à 2 %.

Les différentes compositions cosmétiques ou dermatologiques mentionnées ci-dessus peuvent être obtenues selon les méthodes usuelles utilisées dans ce domaine.

Les compositions cosmétiques selon l'invention peuvent se présenter sous toutes les formes utilisées en cosmétologie : crème ou gel en pots ou en tubes, lait, en flacons de verre ou de matière plastique et éventuellement en flacon-doseur, ampoule.

Les compositions dermatologiques telles que définies ci-dessus, peuvent se présenter sous forme de préparation liquide ou solide à usage topique et notamment sous une des formes suivantes :

- des gels gras,
- des émulsions simples, eau dans huile,
- des émulsions simples, huile dans eau,
- des émulsions multiples, par exemple une émulsion triple eau dans huile dans eau ou huile dans eau dans huile,
- des émulsions complexes contenant des cristaux liquides formant des bicouches lipidiques entourant des phases grasses,
- une émulsion huile dans eau contenant des cristaux liquides,
- des micro-émulsions huile dans eau ou eau dans huile,
- des émulsions contenant des phases huileuses dispersées, différentes et insolubles entre elles,
- des pseudo-émulsions ou dispersion d'une phase grasse dans une phase aqueuse et stabilisée avec des gélifiants tels que le Lubragel (R) (polyglycérylméthacrylate commercialisé par Sederma, France), du Pemulen (R) (carbomères modifiés), de l'Hypan (R), de la gomme Xanthane,de la CMC, de l'hydroxyéthyl cellulose, de l'Amigel (R), de la Polyvinylpyrrolidone, de l'Amercell HM1500 (R), ou un mélange de deux ou plusieurs de ces gélifiants, sans tensio-actifs traditionnels.

La présente invention a également pour objet l'application des phospholipides actifs tels que définis ci-dessus et notamment sous forme de phosphatidyléthanol actif, à la préparation de compositions cosmétiques

ou dermatologiques telles que définies ci-dessus, destinées à être administrées sur la peau.

L'utilisation de ces phospholipides actifs dans des préparations à usage cosmétique ou pharmaceutique a pour but l'apport sur la peau d'organismes vivants, de substances actives par l'intermédiaire des phospholipides actifs définis ci-dessus.

La présente invention a encore pour objet une méthode de traitement cosmétique de la peau sèche ou desséchée caractérisée en ce que l'on applique sur la peau une quantité suffisante d'une composition cosmétique telle que définie ci-dessus.

La présente invention a notamment pour objet une méthode de traitement cosmétique des comédons caractérisée en ce que l'on utilise les compositions cosmétiques telles que définies ci-dessus.

Les exemples suivants illustrent l'invention sans toutefois la limiter.

**EXEMPLE 1 :**

**PREPARATION D'UN PHOSPHOLIPIDE VECTEUR DE VITAMINE A ACIDE**

La transformation d'un mélange de phospholipides extraits de source naturelle en composé vecteur de vitamine A acide est réalisée par un procédé en trois étapes :

1 - Extraction des phospholipides naturels et leur transformation en un composé unique,

2 - Hydrolyse sélective en position 2,

3 - Réacylation du lyso-phospholipide.

On utilisera les abréviations suivantes :

PC : phosphatidylcholine :

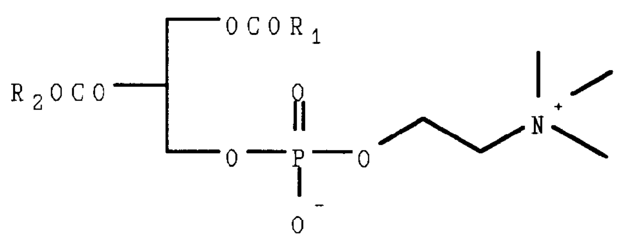

PE : phosphatidyléthanolamine :

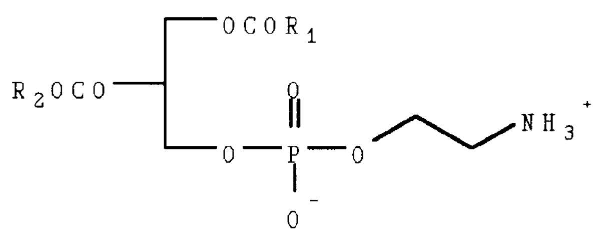

PET : phosphatidyléthanol :

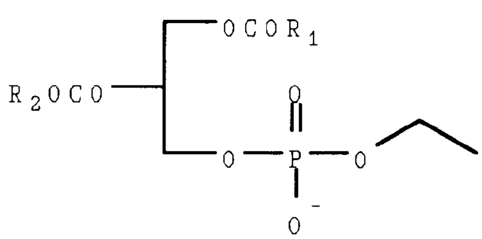

dans laquelle $R_1$ et $R_2$ ont la signification indiquée ci-dessus.

1 - Extraction des phospholipides naturels et leur transformation en un composé unique.

a) Extraction des phospholipides naturels La source de phospholipides utilisée est le jaune d'oeuf de poule. On a utilisé des oeufs du commerce ou du jaune d'oeuf préparé industriellement. A partir des

oeufs du commerce, l'étape préalable est leur durcissement réalisé à environ 100°C, pendant environ 10 mn. Les jaunes sont ensuite récupérés et extraits soit directement soit après séchage.

L'extraction est réalisée en premier lieu par de l'acétone (3 x 200 ml pour 400 g de jaunes humides) par simple agitation du jaune écrasé dans le solvant. La suspension est filtrée et le solide retraité par l'acétone. Le solide résiduel, blanc crème, est séché puis dispersé dans 3 x 200 ml d'éthanol. Après filtration, la phase éthanolique est soit conservée dans le volume nécessaire pour l'étape suivante, soit évaporée sous pression réduite. Le résidu est dilué dans un minimum de dichlorométhane (DCM) et on ajoute 500 ml d'acétone. Les phospholipides précipitent. On garde la suspension à 4°C pendant 16 heures puis on filtre et sèche.

Le bilan de l'extraction est le suivant :

on obtient en moyenne 1 g de phospholipides par jaune d'oeuf et 150 g par kilo de produit sec. Le mélange obtenu est constitué très majoritairement de PC et de PE dans les proportions de 4 pour 1.

b) Transformation du mélange PC/PE en PET

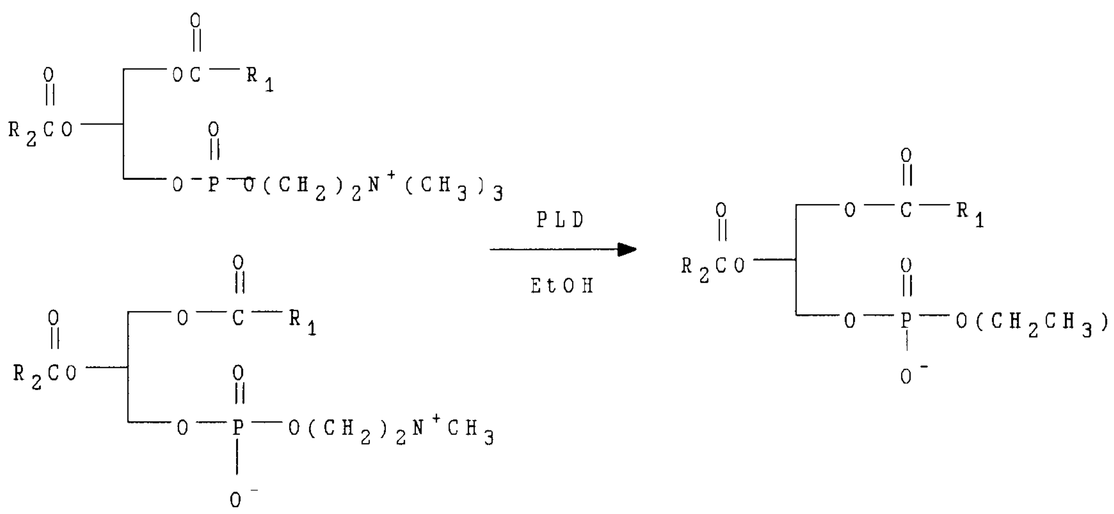


dans lesquels $R_1$ et $R_2$ ont les significations indiquées ci-dessus.

Cette transformation est effectuée en utilisant la propriété de la phospholipase D (PLD) de catalyser les réactions de transphosphatidylation qui permettent de modifier la tête polaire des phospholipides.

La PLD est extraite de feuilles de chou : les feuilles blanches (1 kg) sont hachées à l'aide d'un "blender" avec 1 1 d'eau distillée. Le broyat est filtré et le filtrat (1,1 à 1,2 l) est utilisé tel quel ou dilué. On ajoute 40mM de chlorure de calcium. Le pH est compris entre 5 et 6.

On ajoute à un volume d'extrait enzymatique un demi volume d'une solution éthanolique du mélange PC/PE en quantité variable de 10 à 80 g/l, de préférence 40 g/l et on agite vigoureusement. L'avancement de la réaction est suivi par chromatographie sur couche mince (CCM). Après un temps variant de 2 à 10 h, on observe la disparition du PC et du PE et la formation d'un précipité gommeux. La phase aqueuse est éliminée. Le précipité est dissous dans le minimum de dichlorométhane ou autre solvant convenable, filtré puis purifié par précipitation dans 400 ml d'acétone sous agitation. On filtre et sèche. On obtient avec un rendement de 60 à 90%, un produit blanc crème qui est le sel de calcium de phosphatidyléthanol.

2 - Hydrolyse en position 2 du PET pour obtenir le lyso-PET.

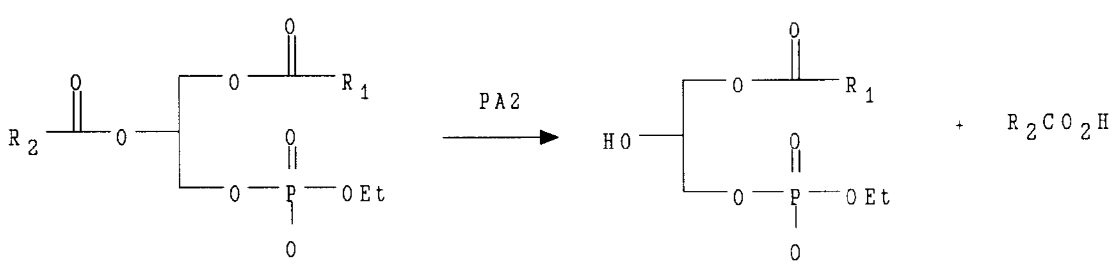


dans lesquels $R_1$ et $R_2$ ont les significations indiquées ci-dessus.

La source utilisée de PA2 est la poudre pancréatique de porc(Fluka). On dissout 2 g de poudre enzymatique dans 10 à 20 ml d'une solution d'acide borique (50 mM) et de chlorure de calcium (50 mM). On amène le pH entre 2 et 3 pendant 20 mn puis ramène le pH entre 7 et 8 avec de la soude 1N. On ajoute alors une solution de 10 g de PET sous forme de sel de calcium (16 mmol) dans 100 à 200 ml d'éther et on maintient le mélange sous une vigoureuse agitation. L'avancement de la réaction est suivie par chromatographie sur couche mince. On observe une disparition régulière du PET au profit du lyso-PET et des acides gras libérés par l'hydrolyse. En fin de réaction, on filtre sur célite. Afin de purifier par précipitation le lysoPET, on évapore l'éther de sorte qu'il ne reste que le volume suffisant à la solubilité de la solution et cette solution est versée dans 400 ml d'acétone sous une vigoureuse agitation. Le lysoPET sous forme de sel de calcium, de couleur blanc crème précipite alors que les acides gras libérés sont solubles dans l'acétone. On filtre et sèche. Le dérivé lysoPET est obtenu avec un rendement de 60 à 90%.

3 - Réacylation de la position 2.

Cette étape est divisée en deux sous-étapes :

- Acidification du lyso-PET,
- Acylation proprement dite après éventuellement l'obtention in situ d'un anhydride de la vitamine A acide.

a) Acidification du lyso-PET.

Le lyso-PET précédemment obtenu contient des formes salines. L'obtention de la forme acide est réalisée par dissolution du produit (5 g) dans un mélange chloroforme ou dichlorométhane (10 ml), méthanol (12 ml) et lavage par de l'acide chlorhydrique 1N (3 x 10 ml). La phase organique est ensuite lavée avec de l'eau distillée et évaporée sous pression réduite en présence de toluène. Le produit brut est séché sous pression réduite dans un dessicateur.

b) Acylation proprement dite après éventuellement l'obtention in situ d'un anhydride de la vitamine A acide.

L'acylation du lyso-PET, forme acide, est réalisée en utilisant l'acide que l'on veut introduire sous forme activée. Pour les acides de hautes valeurs tels que l'acide rétinoïque ou des acides polyinsaturés, on peut soit préparer un anhydride mixte avec l'acide pivalique, soit utiliser dans le milieu réactionnel du dicyclohexylcarbodiimide (DCC). Dans le cas où l'on prépare un anhydride mixte, c'est le groupe acyle de l'anhydride mixte le moins encombré stériquement qui se transfère sur l'alcool accepteur.

Acylation par l'anhydride

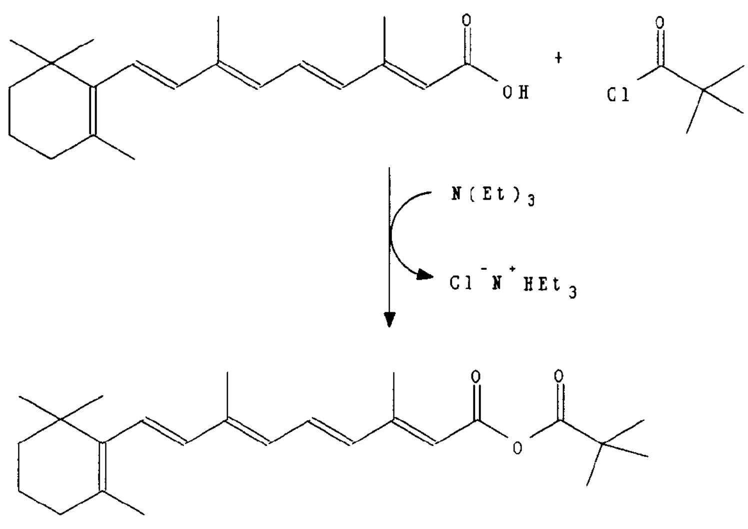


Préparation de l'anhydride : on ajoute à une solution de vitamine A acide (500 mg, 1,66 mmol) dans 20 ml de toluène et d'éther éthylique contenant 1 ml de triéthylamine (7,2 mmol), du chlorure de pivaloyle (0,2 ml, 1,6 mmol). La réaction est effectuée à l'abri de la lumière et sous atmosphère inerte. Le contrôle par CCM indique la disparition de l'acide en quelques minutes.

- Acylation

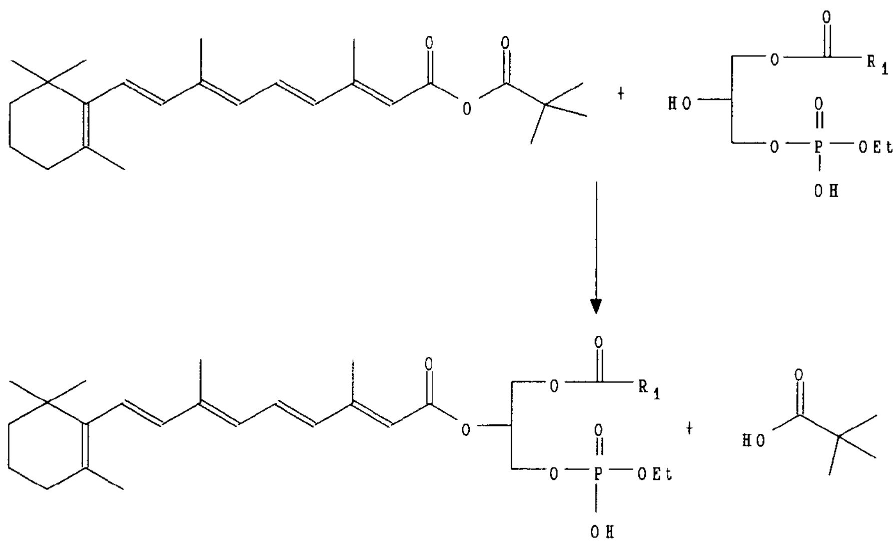

dans lesquels $R_1$ a la signification indiquée ci-dessus.

On ajoute alors le lyso-PET (600 mg, 1,3 mmol) dissous dans 10 ml de toluène et d'éther éthylique et de la 4-diméthylaminopyridine (12 mg, 0,13 mmol) dans 1 ml de toluène ou d'éther éthylique. L'évolution de la réaction est suivie par CCM. La réaction est terminée (disparition du lyso-PET) au bout de 6 à 10 h. On filtre sur célite. On ajoute de l'acide chlorhydrique 1N (10 ml) et récupère la phase organique qu'on lave avec de l'eau. On évapore en présence de toluène. Le mélange brut est fractionné par chromatographie sur silice. Le rendement après purification est de 60%. On obtient ainsi 600 mg de PET-vitamine A.

- Acylation par le dicyclohexylcarbodimiide (DCC)

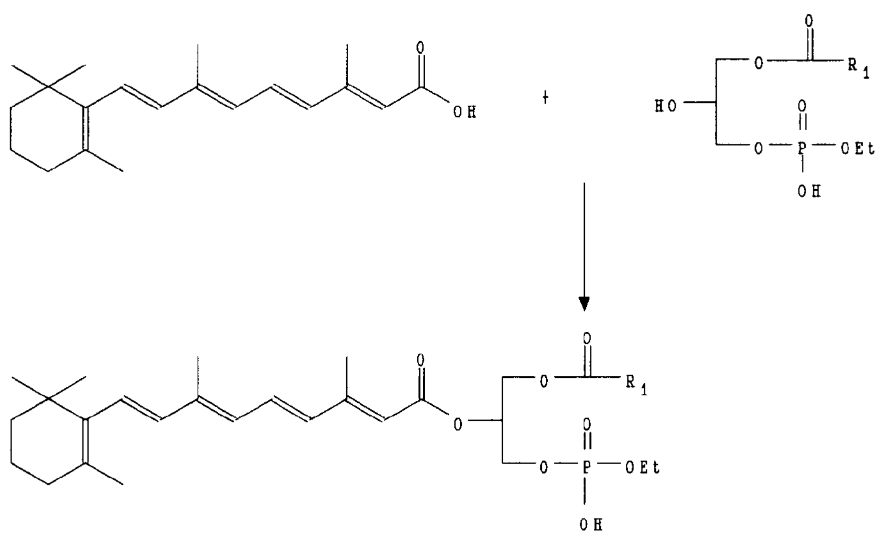

On ajoute à une solution de vitamine A acide (500 mg, 1,66 mmol) dans 20 ml de toluène ou d'éther éthylique contenant 1 ml de triéthylamine (7,2 mmol) le lysoPET (600 mg, 1,3 mmol) dissous dans 10 ml de toluène et d'éther éthylique et de la 4-diméthylaminopyridine (12 mg, 0,13 mmol) dans 1 ml de toluène ou d'éther éthylique et du dicyclohexylcarbodiimide (322 mg,1,56 mmol). L'évolution de la réaction est suivie par CCM. La réaction est terminée (disparition du lyso-PET) au bout de 6 à 10 h. On filtre sur célite. On ajoute de l'acide chlorhydrique 1N (10 ml) et récupère la phase organique qu'on lave avec de l'eau. On évapore en présence de toluène. Le mélange brut est fractionné par chromatographie sur silice. Le rendement après purification est de 60%. On obtient ainsi 600 mg de PET-vitamine A.

- **EXEMPLE 2: Crème ANTI-ACNE**

On chauffe à 70°C la phase grasse suivante :

- Stéaramidopropyl PG-dimonium chloride Phosphate (nom CTFA) 3,0 g
- Cocamidopropyl PG-dimonium chloride Phosphate (nom CTFA) 1,0 g

- Alcool cétylique 3,0 g
- Myristyl myristate 5,0 g
- Polyisobutene hydrogéné 2,0 g
- Beurre de karité 2,0 g
- Stéarate de Propylene glycol 3,0 g
- Huile de silicone 2,0 g
- Huile végétale 5,0 g
- Anti-oxydant 0,2 g
- Acétate d'oléyle 1,0 g

On prépare par ailleurs la phase aqueuse suivante et on la chauffe à 70°C :

- Eau déminéralisée QSP 100,0 g
- Glycérine 10,0 g
- Hydroxy éthylcellulose modifiée 0,5 g
- PVP 1,0 g
- Conservateurs 0,52 g

On réalise l'émulsion huile dans eau (H/E) en mélangeant vivement les deux phases à 90°C pendant 10 minutes. On refroidit ensuite lentement sous agitation modérée. A 45°C, on ajoute 0,5 % de parfum, puis 0,5 % de all-trans Rétinoyl-2 Phosphatidyl Ethanol.

On continue ensuite à refroidir jusqu'à 25°C.

Cette émulsion H/E cationique sera utilisée dans le traitement de l'acné.

- **EXEMPLE 3: Crème pour le traitement des peaux atopiques**
  - γ linolényl Phosphatidyl Ethanol 5,0 g
  - Alcoyle phosphate de potassium 2,0 g
  - Palmitate d'éthyl hexyle 8,0 g
  - Lanoline hydrogénée 5,0 g
  - Triglycérides d'acides gras 4,0 g
  - Stéarate de sorbitan 1,0 g
  - Polymère carboxyvinylique neutralisé 0,4 g
  - Conservateurs 0,4 g
  - Eau purifiée QSP 100,0 g
- **EXEMPLE 4 : Crème pour le traitement du psoriasis**
  - EPA-2 Phosphatidyl Ethanol 1,0 g
  - DHA-2 Phosphatidyl Ethanol 1,0 g
  - Stéarate de glycérol 4,0 g
  - Palmitate de sorbitan 6,0 g
  - Perhydrosqualène 5,0 g
  - Diisopropyl-cyclohexane 7,0 g
  - Triglycérides capriques/capryliques 9,0 g
  - Glycérine 5,0 g
  - Conservateurs 0,35 g
  - Eau purifiée QSP 100,0 g
- **EXEMPLE 5 : Lait solaire**
  - Ascorbyl-2 Phosphatidyl éthanol 3,0 g
  - Filtres solaires 5,0 g
  - Huile de vaseline 10,0 g
  - Cétéaryl octanoate 4,0 g
  - Phospholipides de soja déhuilé 5,0 g
  - Huile de silicone 2,5 g
  - Ether cétylique P.O.E. 2,0 g
  - Stéarate de sorbitan 1,0 g
  - Conservateurs 0,35 g
  - Composition aromatique 0,5 g
  - Eau purifiée QSP 100,0 g
- **EXEMPLE 6 : Emulsion multiple pour le traitement du vieillissement actinique**

On chauffe à 80°C la phase aqueuse suivante, dite phase aqueuse interne :

- Eau déminéralisée 26,52 g
- Méthylparaben 0,1 g
- Sulfate de magnésium 0,28 g

- Glycérine 30' B 0,8 g
- O-cymen-5-ol 0,04 g

On chauffe séparément la phase grasse suivante :

- Glycéryl isostéarate 2,0 g
- Huile de ricin hydrogénée polyoxyéthylénée (7 moles) 0,2 g
- Huile de soja 8,2 g
- Propylparaben 0,06 g
- Huile de silicone volatile 1,6 g
- Lactyl-2 Phosphatidyl éthanol 5,0 g

On disperse la phase aqueuse dans la phase grasse à 80°C en agitant vigoureusement 5 minutes. Puis on refroidit lentement jusqu'à 25°C.

On disperse ensuite cette émulsion primaire eau/huile dans la phase aqueuse suivante, dite phase aqueuse externe, en mélangeant doucement à température ambiante :

- Eau déminéralisée QSP 100,0 g
- Lubragel MS (R) 15,0 g
- Carbopol 980 (R) 0,03 g
- Tétrasodium EDTA 0,054 g
- Méthylparaben 0,216 g
- Imidazolidinyl urée 0,216 g
- Hydroxyde de sodium pur 0,125 g

**EXEMPLE 7 : Emulsion à phases gémellaires cicatrisante**

On chauffe à 80°C la phase grasse suivante :

- Alcool stéarylique 1,0 g
- Alcool cétylique 2,0 g
- Cétéaryl octanoate 4,0 g
- Polysorbate 60 4,0 g
- Sorbitan stéarate 4,0 g
- Huile de carthame 6,0 g
- Beurre de karité 3,0 g
- Huile de silicone 0,5 g
- Tocophérols 0,05 g
- Asiaticosyl-2 Phosphatidyl Ethanol 0,5 g
- Madecassyl-2 Phosphatidyl Ethanol 0,5 g

On chauffe à 80°C la phase aqueuse suivante :

- Eau déminéralisée QSP 100,0 g
- Polymère carboxy vinylique 0,3 g
- Conservateur 0,7 g
- Lubragel MS (R) 5,0 g
- Hydroxyde de sodium pur 0,3 g

On disperse la phase grasse dans la phase aqueuse et on agite vigoureusement pendant 10 minutes. On refroidit ensuite lentement l'émulsion ainsi formée jusqu'à 25°C.

**Exemple 8 : Emulsion eau/silicone pour le traitement des kératoses solaires**

On chauffe à 60°C la phase grasse suivante :

- Eau déminéralisée QSP 100,0 g
- Chlorure de sodium 0,8 g
- Acide citrique pur 0,01 g
- Méthylparaben 0,25 g
- Propylène glycol 2,0 g
- O-cymen-5-ol 0,1 g

On chauffe à 60°C la phase silicone suivante :

- Stéarate d'isocétyle 3,0 g
- Arlacel 83 (R) 0,8 g
- Huile de ricin hydrogénée 0,3 g
- Elfacos ST9 (R) 2,0 g

- NDGA-2 Phosphatidyl Ethanol 2,0 g
- Silicone DC 3225 (R) (DOW CORNING) 9,0 g
- Silicone volatile 4,0 g

On disperse la phase aqueuse dans la phase silicone sous agitation moyenne pendant 10 minutes. On refroidit ensuite l'émulsion ainsi formée jusqu'à 25°C.

**EXEMPLE 9 : Emulsion sans émulsionnant précurseur de bronzage**

On chauffe à 80°C la phase grasse suivante :

- Huile de germe de blé 4,0 g
- Polyisobutène 4,0 g
- Octyl stéarate 4,0 g
- Céramide HO3 2,0 g
- Tyrosinyl-2 Phosphatidyl éthanol 2,0 g

On chauffe à 80°C la phase aqueuse suivante :

- Glycérine 30' codex 3,0 g
- Polymère carboxyvinylique 0,45 g
- Lubragel MS (R) 4,0 g
- Hydroxyde de sodium pur 0,055 g
- Conservateurs 0,55 g
- Parfum 0,20 g
- Eau déminéralisée 60,0 g

On disperse la phase grasse dans la phase aqueuse sous une très faible agitation et cisaillement élevé pendant 1/2 heure. On refroidit ensuite lentement l'émulsion ainsi formée jusqu'à 45°C, on ajoute alors sous bonne agitation :

- Talc 3,0 g

Quand la dispersion du talc est bien terminée, on continue à refroidir sous agitation lente. Quand la température est de 25°C, on ajoute sous agitation modérée :

- Parfum 0,2 g

**EXEMPLE 10 : Emulsion anti-inflammatoire**

On prépare une émulsion huile dans eau de la manière suivante.

On chauffe à 80°C la phase grasse suivante :

- Stéarate de glycérol autoémulsionnable (arlacel 165 (R) de la Sté ICI) 6,0 g
- Alcool cétylique 1,0 g
- Stérol de soja éthoxylé (générol 122 E 10 (R) de la Sté Henkel) 2,0 g
- Mélange d'huile de vaseline et d'alcool de lanoline (amerchol L101 (R) de la Sté Amerchol) 3,0 g
- Pétrolatum et alcool de lanoline (amerchol CAB (R) de la Sté Amerchol) 1,0 g
- Huile d'olive 6,0 g
- Beurre de karité 3,0 g
- Propyl paraben 0,05 g
- Glycyrrhétinyl-2 Phosphatidyl Ethanol 1,0 g

On chauffe à 80°C la phase aqueuse suivante :

- Eau déminéralisée 60,0 g
- Sorbitol à 70 % 3,0 g
- Gomme xanthane 0,3 g
- Méthylparaben 0,1 g

Quand la gomme xanthane est bien dispersée, on ajoute la phase grasse à la phase aqueuse à 80°C, et on agite vivement pendant 20 minutes. L'émulsion se forme.

Ensuite on réduit l'agitation et on refroidit lentement l'émulsion jusqu'à 40°C.

On y ajoute alors 2 g d'eau contenant 0,15 g d'imidazolidinyl urée, puis 0,3 g de parfum.

**TEST D'ACTIVITE D'UN PHOSPHOLIPIDE ACTIF**

A titre d'exemple, on a testé le pouvoir comédolytique du all-trans rétinoyl-2 phosphatidinyl éthanol suivant le protocole ci-après :

L'animal choisi pour le test d'activité comédolytique est la souris Hairless Rhino (hr rh), de sexe femelle, ce choix étant dû au fait que la peau d'un tel animal présente une grande densité de comédons de large diamètre et d'orifice étroit.

L'application d'agents comédolytiques sur la peau de l'animal provoque l'ouverture de l'orifice du comédon, la libération du matériel corné et du sébum qu'il contient.

Deux lots sont constitués, chacun des lots comportant 6 souris âgées de 6 semaines au début de l'essai et pesant en moyenne 18 grammes chacune.

Le premier lot est constitué de souris traitées avec de l'eau distillée (lot témoin négatif), le second lot est constitué de souris traitées avec le produit à étudier, un tel traitement consistant en une application topique du produit étudié sur la zone interscapsulaire à la dose de 0,02 ml, 5 jours sur 7, pendant 21 jours.

Les animaux sont sacrifiés à l'issue des trois semaines de traitement, 24 heures après la dernière application.

Des biopsies de la peau sont alors prélevées au niveau des zones traitées des animaux et à partir de ces biopsies, des coupes sont préparées, en vue d'une étude morphométrique classique réalisée selon les méthodes connues de l'homme de métier.

Les paramètres suivants sont mesurés :
diamètre de l'ouverture de comédon à la surface soit d
diamètre du comédon soit D
profil comédonien soit R = d/D
Le rapport R = d/D permet de quantifier l'action des agents comédolytiques.

Le pourcentage d'inhibition des comédons est calculé pour le produit à étudier par rapport au témoin négatif, soit le rapport suivant :

$$\% \text{ inhibition} = \frac{(\text{R produit} - \text{R témoin négatif}) \times 100}{\text{R témoin négatif}}$$

L'activité comédolytique du all-trans rétinoyl-2 phosphatidinyl éthanol à 0,1 % en solution dans l'éthyldiglycol a été mesurée suivant cette méthode et a donné les résultats suivants :

- R Produit = 0,89
- R Témoin négatif = 0,78
- % inhibition = 14,1 %

On voit donc que le all-trans rétinoyl-2 phosphatidinyl éthanol à la dose de 0,1 % a une bonne activité comédolytique.

**Revendications**

**1)** Phospholipides actifs de formule (I) :

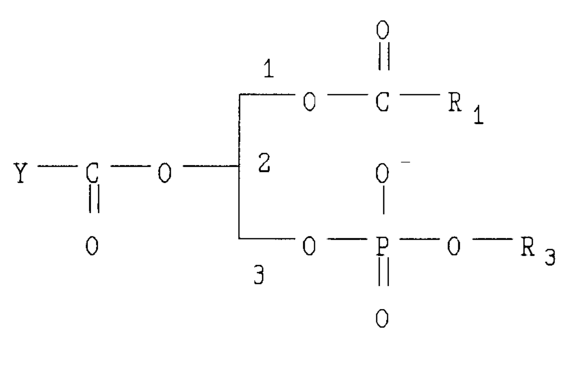

**(I)**

dans laquelle :
$R_1$ représente notamment une chaîne aliphatique de 14 à 24 atomes de carbone, saturée ou avec 1 ou 2 insaturations,
$R_3$ représente notamment un reste de choline, d'éthanolamine, de glycérol, de sérine, d'inositol, d'éthanol, de n-propanol, de n-butanol ou encore d'éthylène glycol,
et Y est tel que

Y-C-
||
O

représente une molécule active en position 2 du glycérol destinée à être libérée sous l'action de phospholipases.

**2)** Phospholipides actifs, tels que définis à la revendication 1, caractérisés en ce que $R_3$ représente un radical éthyle.

**3)** Phospholipides actifs, tels que définis à la revendication 1, caractérisés en ce que la molécule active

Y-C-
||
O

en position 2 est choisie parmi les molécules suivantes : la vitamine A acide, l'acide all-trans rétinoïque, l'acide 9-cis rétinoïque, l'acide 13-cis rétinoïque, les acides gras essentiels comme l'acide γ linolénique, l'acide α linolénique, l'acide eicosapentaenoïque (EPA), l'acide docosahexaenoïque (DHA), les acides α hydroxylés comme l'acide glycolique, l'acide lactique, l'acide tartrique, l'acide α méthyllactique, l'acide α hydroxybutyrique, l'acide gluconique, l'acide mandélique, l'acide mucique, l'acide malique, l'acide α phényllactique, l'acide saccharique, l'acide tartronique ; les acides divers tels que l'acide kojique, l'acide asiaticoside, l'acide madécassique, l'acide benzoïque, l'acide glutamique, l'acide malonique, l'acide phytique, l'acide ascorbique, l'acide nordihydroguaiaretique, l'acide salicylique, l'acide 18β glycyrrhétinique ; les acides aminés comme la tyrosine, l'hydroxyproline, la lysine, l'arginine, des petits peptides fonctionnels comme le pyroGlu-Glu-Asp-Ser-GlyoH ou le Gly-His-Lys ou le Arg-Gly-Asp-Ser, ou encore les monoesters de diacide tels que le succinate de farnésile, de rétinol et les monoamides de diacide de formule générale

$$HO_2\text{-}C\text{-}(CH_2)_n\text{-}CO\text{-}X\text{-}R$$

dans laquelle n représente un nombre compris entre 2 et 16, X représente un atome de soufre, d'azote ou d'oxygène et R représente un radical inclus dans la liste des acides ci-dessus.

**4)** Phospholipides actifs, tels que définis aux revendications 1 à 3, caractérisés en ce que la molécule active

Y-C-
||
O

en position 2 est choisie parmi les substances suivantes : la vitamine A acide, l'acide γ linolénique, l'acide eicosapentaenoïque (EPA), l'acide docosahexaenoïque (DHA), l'acide kojique, l'acide asiaticoside, l'acide madécassique, l'acide glutamique, l'acide phytique, l'acide ascorbique, l'acide lactique, l'acide glycolique, l'acide nordihydroguaiaretique, les acides aminés comme la tyrosine, des petits peptides fonctionnels comme le pyroGlu-Glu-Asp-Ser-GlyoH ou le Gly-His-Lys ou le Arg-Gly-Asp-Ser, l'acide 18β glycyrrhétinique.

**5)** Procédé de préparation de phospholipides actifs de formule (I) telle que définie aux revendications 1 à 4, caractérisé en ce que l'on soumet un phospholipide ou un mélange de phospholipides de sources naturelles, de formule (II) :

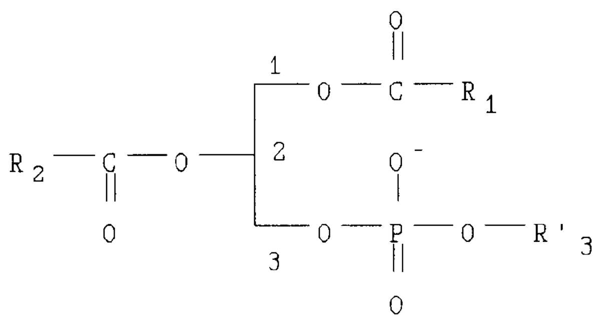

(II)

dans laquelle $R_1$ et $R_2$ identiques ou différents représentent une chaîne aliphatique de 14 à 24 atomes de carbone saturée ou avec 1 ou 2 insaturations, $R'_3$ représente un reste de choline, d'éthanolamine, de glycérol, de sérine ou encore d'inositol, à une réaction de substitution de $R'_3$ par un radical éthyle, propyle ou butyle au moyen d'une transphosphatidylation enzymatique pour obtenir un produit de formule (III) :

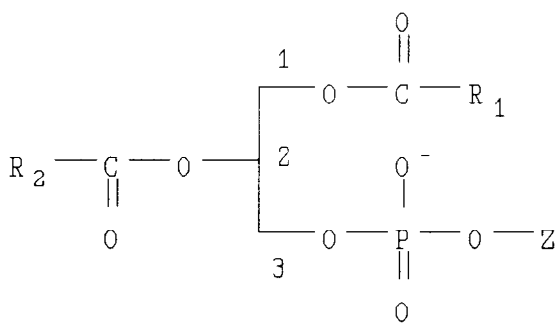

(III)

dans laquelle $R_1$ et $R_2$ ont les significations indiquées ci-dessus et Z représente un radical éthyle, propyle ou butyle, que l'on soumet à une hydrolyse par voie enzymatique de la fonction ester en position 2 du glycérol, pour obtenir un produit de formule (IV) :

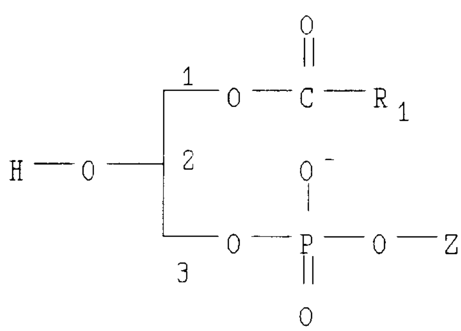

(IV)

dans laquelle $R_1$ et Z ont les significations indiquées ci-dessus, que l'on soumet à une réaction d'acylation de l'hydroxyle libéré, par un anhydride de formule (V) :

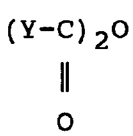

(V)

ou un anhydride mixte correspondant dans laquelle

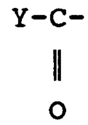

représente un reste de molécule active telle que définie aux revendications 1, 3 et 4.

**6)** Procédé de préparation de phospholipides actifs tel que défini à la revendication 5, caractérisé en ce que :

- les sources naturelles de phospholipides sont le jaune d'oeuf, la lécithine végétale,
- le phospholipide de source naturelle est la phosphatidylcholine ou la phosphatidyléthanolamine ou un mélange de ces phospholipides,
- la substitution du radical $R'_3$ par un radical éthyle, propyle ou butyle, effectuée par transphosphatidylation enzymatique est réalisée au moyen de la phospholipase D en milieu éthanolique, propanolique ou butanolique, cette réaction étant complète et univoque ;
- l'hydrolyse par voie enzymatique pour obtenir le lysophosphatidyl éthanol de formule (IV) est réalisée au moyen de la phospholipase $A_2$ enzyme utilisée non purifiée, en milieu calcique $Ca^{++}$ ;
- l'acylation de l'hydroxyle libéré dans le composé de formule (IV) par la ou les molécules biologiquement actives à transporter dans les membranes est réalisée par voie enzymatique ou chimique, après acidification du lysophosphatidyl éthanol (propanol ou butanol), en utilisant un anhydride simple ou mixte de la substance active, au sein d'un solvant tel que par exemple le diéthyléther ou le toluène.

**7)** Procédé de préparation de phospholipides actifs tel que défini aux revendications 5 et 6, caractérisé en ce que la substance active à incorporer au phospholipide en position 2 est choisie parmi les substances définies à la revendication 3 et de préférence celles définies à la revendication 4.

**8)** Compositions cosmétiques ou dermatologiques destinées au soin et traitement de la peau et plus particulièrement aux peaux acnéiques, aux peaux déshydratées, aux peaux lésées, aux peaux ridées, caractérisées en ce qu'elles renferment des phospholipides actifs tels que définis aux revendications 1 à 4 et des excipients usuels.

**9)** Compositions cosmétiques ou dermatologiques selon la revendication 8, destinées notamment au soin des comédons, caractérisées en ce qu'elles renferment un phospholipide actif vecteur d'un ester de rétinol tel que la vitamine A acide ou l'un de ses isomères.

**10)** Compositions cosmétiques ou dermatologiques, telles que définies à la revendication 8 ou 9, caractérisées en ce qu'elles renferment un ou plusieurs principes actifs liposolubles complémentaires choisis notamment parmi le palmitate de vitamine A, l'acide linoléique des filtres solaires liposolubles, des insaponifiables d'origine végétale, un mélange huileux contenant de l'acide ximénique, de l'extrait essentiel d'huile de sésame, de l'huile de maïs peroxydée, de l'acétate de tocophérols, des tocophérols naturels, du farnesol.

**11)** Compositions dermatologiques ou cosmétiques, telles que définies à la revendication 8 ou 9, caractérisées en ce qu'elles renferment un ou plusieurs principes actifs hydrosolubles complémentaires choisis notamment parmi le lactate de sodium, des extraits de biolysat d'Hafnia, des extraits de biolysat de Klebsiella pneumoniae et des filtres solaires hydrosolubles.

**12)** Application des phospholipides actifs tels que définis aux revendications 1 à 4, à la préparation de compositions cosmétiques ou dermatologiques telles que définies à l'une quelconque des revendications 8 à 11.

**13)** Méthode de traitement cosmétique de la peau sèche ou desséchée caractérisée en ce que l'on applique sur la peau une quantité suffisante d'une composition cosmétique telle que définie à l'une quelconque des revendications 8 à 11.

**14)** Méthode de traitement cosmétique des comédons caractérisée en ce que l'on utilise les compositions cosmétiques telles que définies à la revendication 9.

Office européen des brevets

# RAPPORT DE RECHERCHE EUROPEENNE

Numero de la demande
EP 94 40 2975

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | US-A-4 882 165 (C. ANTHONY HUNT)<br>* le document en entier *<br>--- | 1,3,4 | C07F9/10<br>A61K7/48<br>C12P9/00<br>C07F9/117<br>C07K7/06<br>C07K5/08<br>C07K5/10 |
| X | J. CHROMATOGR. (JOCRAM,00219673);89; VOL.364 (2); PP.397-408, CZECH. ACAD. SCI.;INST. MICROBIOL.; PRAGUE; 142 20 (CS) Rezanka T. 'Preparative separation of algal'<br>* le document en entier *<br>--- | 1,3,4 | |
| X | AGRIC. BIOL. CHEM. (ABCHA6,00021369);90; VOL.54 (1); PP.191-5, OKAYAMA UNIV.;FAC. AGRIC.; TSUSHIMA; 700; JAPAN (JP) Sekiya J et al 'Molecular species of phospholipids of Lilium longiflorum pollen'<br>* le document en entier *<br>--- | 1,3,4 | |
| X | BIOCHEMISTRY (BICHAW,00062960);90; VOL.29 (37); PP.8548-54, UNIV. HELSINKI;DEP. MED. CHEM.; HELSINKI; 00170; FINLAND (FI) Kasurinen J et al 'Affinity of phosphatidylcholine molecular species for the bovine phosphatidylcholine and phosphatidylinositol transfer proteins. Properties of the sn-1 and sn-2 acyl binding sites'<br>* le document en entier *<br>---<br>-/-- | 1,3,4 | DOMAINES TECHNIQUES RECHERCHES (Int.Cl.6)<br>C07F<br>C07K<br>A61K |

Le présent rapport a été établi pour toutes les revendications

| Lieu de la recherche | Date d'achèvement de la recherche | Examinateur |
|---|---|---|
| LA HAYE | 21 Mars 1995 | Beslier, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................
& : membre de la même famille, document correspondant

EPO FORM 1503 03.82 (P04C02)

Office européen des brevets

# RAPPORT DE RECHERCHE EUROPEENNE

Numero de la demande
EP 94 40 2975

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | J. LIPID RES. (JLPRAW,00222275);92; VOL.33 (6); PP.879-87, BOWMAN GRAY SCH. MED.;DEP. COMP. MED.; WINSTON-SALEM; 27157-1040; NC; USA (US)<br>Parks J S et al 'Inhibition of lecithin:cholesterol acyltransferase activity by nthetic phosphatidylcholine species containing eicosapentaenoic acid or docosahexaenoic acid in the sn-2 position'<br>* le document en entier *<br>--- | 1,3,4 | |
| X | CHEMICAL ABSTRACTS, vol. 116, no. 6, 10 Février 1992, Columbus, Ohio, US; abstract no. 046304,<br>HIBINO H ET AL 'Infusion solutions containing stable lipids with highly unsaturated fatty acids'<br>* abrégé *<br>& JP-A-91 153 628 (NIPPON OIL AND FATS CO., LTD.;JAPAN ) 1 Juillet 1991<br>--- | 1,3,4 | DOMAINES TECHNIQUES RECHERCHES (Int.Cl.6) |
| X | CHEMICAL ABSTRACTS, vol. 113, no. 5, 30 Juillet 1990, Columbus, Ohio, US; abstract no. 034703,<br>HIBINO H ET AL 'Antiallergy agents containing highly unsaturated diacyl glycerols'<br>* abrégé *<br>& JP-A-9 045 424 (NIPPON OILS AND FATS CO., LTD.;JAPAN ) 15 Février 1990<br>---<br>-/-- | 1,3,4 | |

Le présent rapport a été établi pour toutes les revendications

| Lieu de la recherche | Date d'achèvement de la recherche | Examinateur |
|---|---|---|
| LA HAYE | 21 Mars 1995 | Beslier, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numero de la demande
EP 94 40 2975

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | CHEMICAL ABSTRACTS, vol. 113, no. 19, 5 Novembre 1990, Columbus, Ohio, US; abstract no. 169386, TOKUMURA A ET AL 'Structural analysis of biologically active phospholipids by GC/MS (gas chromatography/mass spectrometry)' * abrégé * & NIPPON IYO MASU SUPEKUTORU GAKKAI KOENSHU (NIMKEN,0916085X);89; VOL.14,; PP.31-8, UNIV. TOKUSHIMA;FAC. PHARM. SCI.; TOKUSHIMA; JAPAN (JP) --- | 1,3,4 | |
| Y | METHODS ENZYMOL. (MENZAU,00766879);81; VOL.72 (LIPIDS, PART D); PP.632-9, MAX-PLANCK INST. BIOPHYS. CHEM.;GOETTINGEN; D-3400; FED. REP. GER. (DE) Eibl H et al 'Preparation of phospholipids and their analogs by ospholipase D' * le document en entier * --- | 1,5-7 | |
| | | | DOMAINES TECHNIQUES RECHERCHES (Int.Cl.6) |
| Y | ARZNEIM.-FORSCH. (ARZNAD,00044172);85; VOL.35 (3); PP.587-92, UNIV. TOKUSHIMA;FAC. PHARM. SCI.; JAPAN (JP) Tokumura A et al 'Effects of lysophosphatidic acids and their structural analogs on arterial blood pressure of cats' * le document en entier * --- | 1,5-7 | |
| | -/-- | | |

Le présent rapport a été établi pour toutes les revendications

| Lieu de la recherche | Date d'achèvement de la recherche | Examinateur |
|---|---|---|
| LA HAYE | 21 Mars 1995 | Beslier, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

Office européen des brevets

RAPPORT DE RECHERCHE EUROPEENNE

Numero de la demande
EP 94 40 2975

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| Y | BIOCHEMISTRY (BICHAW);75; VOL.14 (23); PP.5021-33, MASSACHUSETTS INST. TECHNOL.;DEP. BIOL.; CAMBRIDGE; MASS. Chakrabarti P et al 'New approach to the study of phospholipid-protein interactions in biological membranes. Synthesis of fatty acids and phospholipids containing photosensitive groups'<br>* le document en entier *<br>--- | 1,5-7 | |
| Y | PHARMAZIE (PHARAT,00317144);84; VOL.39 (3); PP.150-3, KARL-MARX-UNIV.;SEKT. BIOWISS.; LEIPZIG; DDR-7010; GER. DEM. REP. (DD)<br>Kertscher H P et al 'Synthese und Charakterisierung einiger saurer Phospholipide mit Alkylkopfgruppen'<br>* le document en entier *<br>--- | 1,5-7 | |
| Y | CHEM. PHYS. LIPIDS (CPLIA4,00093084);89; VOL.50 (2); PP.135-42, UNIV. MILANO;FAC. FARM.; MILAN; ITALY (IT)<br>Sale M F O et al 'Enzymic synthesis and thermotropic behavior of phosphatidylethanol'<br>* le document en entier *<br>--- | 1,5-7 | DOMAINES TECHNIQUES RECHERCHES (Int.Cl.6) |
| Y | EP-A-0 036 583 (MAX-PLANCK-GESELLSCHAFT ZUR FÖRDERUNG DER WISSENSCHAFTEN E.V.)<br>* le document en entier *<br>--- | 1,5-7 | |
| A | EP-A-0 283 713 (INDENA S.P.A.)<br>* le document en entier *<br>--- | 1,8-14 | |
| A | FR-A-2 517 310 (UNILEVER N.V.)<br>* le document en entier *<br>----- | 1,8-14 | |

Le présent rapport a été établi pour toutes les revendications

| Lieu de la recherche | Date d'achèvement de la recherche | Examinateur |
|---|---|---|
| LA HAYE | 21 Mars 1995 | Beslier, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)